# EUROPEAN PATENT APPLICATION

(11) **EP 1 206 931 A1**
(43) Date of publication of application: **22.05.2002**
(21) Application number: 00811079.3
(22) Date of filing: 15.11.2000
(51) Int. Cl.: A61K 7/40

(54) **Chitosan formulations**

(71) Applicant: ERPHAR Société d'Etudes et de Recherches Pharmaceutiques et Cosmétiques, 75007 Paris (FR)
(72) Inventor: Baschong, Werner, 4056 Basel (CH); Roding, Joachim Friedrich, 22765 Hamburg (DE); Fankhauser, Peter, 4107 Ettingen (CH); Falcigno, Pasquale Alfred, 4052 Basel (CH); Mongiat, Sébastien, 68480 Roppentzwiller (FR); Cotte, Jean, 69450 Saint Cyr au Mont d'Or (FR)
(74) Representative: Goulard, Sophie

(57) **Abstract**

Disclosed is a method for (a) the removal of contaminating metal ions from the skin or (b) supplying essential metal ions to the skin, comprising applying to the skin a composition comprising a cosmetically compatible polymer matrix in which (a) chitosan or chitosan derivatives or (b) chitosan or chitosan derivatives which are preloaded with essential metal ions are incorporated and wherein the corresponding polymer matrix is able to immobilize the chitosan and keep it in intimate contact with the skin.

## Description

Chitosan and derivatives thereof and there included phosphonomethylated chitosan (P-chitosan) are polymeric and insoluble in conventional occlusive physiological cosmetical bases and therefore difficult to formulate as a cosmetic skin penetrating active. However, this property allows the formation of a polymeric film on the skin. This intimate contact with the skin is a prerequisite for skin metal control by a chelating agent in order to either remove contaminating metal ions from the skin, or - in the case where the chelating agent is preloaded with essential metal ions - to supply these metal ions to the skin. Within this respect, it is very important, that the active principle is immobile at the skin surface in order to establish the necessary concentration gradient between the skin and the chelator. Such a gradient is difficult to establish with conventional chelators, since due to their small size and high mobility they will penetrate into the skin and thus impede the formation of such a gradient. Conventional chelators are therefore not suitable for such an application.

Surprisingly it was found that chitosan and/or chitosan derivatives when incorporated into a polymer matrix which is able to immobilize the chitosan allow the controlled diffusion of essential metal ions either into or out of based on whether the chelator is preloaded or not with metal ions.

Therefore, the present invention relates to a method for
(a) the removal of contaminating metal ions from the skin or
(b) supplying essential metal ions to the skin
comprising applying to the skin a composition comprising a cosmetically compatible polymer matrix in which (a) chitosan or chitosan derivatives or (b) chitosan or chitosan derivatives which are preloaded with essential metal ions are incorporated and wherein the corresponding polymer matrix is able to immobilize the chitosan and keep it in intimate contact with the skin.
In turn, the chelator has the possibility of establishing a negative or a positive gradient in the direction of the skin surface or the metal ions can diffuse from the active-metal complex to the skin and supply it with such essential metals.

The method of the present invention refers to topical application to the skin. Such forms include dermal and transdermal patches, gels, creams, dispersions, emulsions (water-in-oil or oil-in-water), suspensions, lotions, foams, mousses, masks and the like.

The adhesive dermal or transdermal patch is of simple construction, and is typically comprised of an impermeable backing layer, a single pressure sensitive adhesive, i.e. self-adhesive, layer, the polymer matrix and a removable protective layer.

These components may be suitably selected from known materials in view of safety for the skin, adhesiveness to the skin, and the like.

Dermal or transdermal patches for use in the present invention are nonwoven, preferably elastic materials which include natural rubber-based, synthetic rubber based, silicone-based and acrylic-based materials. Among the synthetic rubber-based materials for example a styrene-isopropene-styrene-block copolymer.

Matrices suitable for the incorporation of chitosan and loading of metal ions are known in the art. They include water insoluble films, foams, or gels. One such film can be made by combining hyaluronic acid with a polyanionic modified polysasccharide as described by US-A-5,017,229.

In a preferred embodiment of the present invention the patch consists of a nonwoven substrate coated with a bioadhesive polymer matrix made mainly by a mixture of adhesive and non-adhesive polymers.

Preferred matrices are:
- hydrophilic adhesive copolymers, for example the copolymer of dimethylaminoethyl methacrylate and neutral methacrylic esters;
- hydrophobic non-adhesive copolymer) : copolymer of ethylacrylate and methylmethacrylate;
- PVM/MA copolymer, anhydrous form, water insoluble but easily dispersible in water
- PVM/MA copolymer, free acid form, water soluble
- PVP/VA Copolymer, water soluble,
- PVP or N-Vinyl pyrrolidone
- PVP/VA Copolymer
- Polyquaternium 37 and Paraffinum Liquidum and PPG-1 Trideceth-6
- Polyquaternium 37 and Propylene Glycol Dicaprylate Dicaprate and PPG-1 Trideceth-6

The patches used in the present invention are prepared by first preparing the polymer solution which constitutes the bioadhesive polymer matrix. In a second step the nonwoven is coated with this bioadhesive to an optimized thickness. The coating machine must contain a reservoir of liquid (bioadhesive polymer matrix) through which the nonwoven substrate is passed.

The coated substrate is then dried to evaporate the solvent and seal the bioadhesive to the fibres of the nonwoven substrate.

Further preferred suitable matrices for the immobilization of chitosan are Hydrogels.

Hydrogels are lightly cross-linked polymer networks that swell in water. At equilibrium their swollen water content is high, ranging form about 30 to more than 99 % b.w.. Hydrogels are unique because of this large amount of "resident" water, which acts as a solute transport medium and as a plastisizer, permitting these materials to behave viscoelastically.

The majority of hydrogels originate from synthetic monomers which yield polymeric architectures with both backbone and pendent groups. Usually the former is aliphatic and relatively hydrophobic, whereas the pendant groups can be hydrophilic and polar such as hydroxyl, hydroxyalkyl, amide or pyrollidone, and may include electrically charged groups such as carboxyl, N-alkylamide or sulphonate. Crosslinking agents such as di(meth)acrylates and epichlorhydrin form covalent chain junctions.

Example of hydrogels are such as those obtained from synthetic polymers such as polyhydroxy-ethylmethycrylate (PHEMA) or from semisynthetic derivatives of natural polysaccharides such as the crosslinked hyaluronic acid derivative with vinyl-sulfone or dextran dialdehyde cross-linked gelatin hydrogel films.

Sugar-based hydrogels as described in Biomaterials 19 (1998), p. 69-76, may find particular use as polymer matrix for the method of the present invention because of their high water content, homogeneity, stability and expected no-toxicity.

Hydrogels can be used in the form of fibers, membranes, threads, gauzes and sponges.

Hydrogels containing chitosans can be prepared by incorporation of the chitosan in the gel and swelling said hydrogel in a dry state in an aqueous solution containing the chitosan.

In a second process the incorporation of the chitosan in the hydrogel material comprises mixing the chitosan into the solution of said hydrogel and than radiating it to obtain the hydrogel.

Other preferred matrices wherein the chitosan or the metal ion loaded chitosan is immobilized are:
- Polyvinyl Alcohol; the high intermolecular cohesion of PVA makes good film-forming property with moisture permeability. PVA adsorbs and desorbs moisture in order to maintain a state of equilibrium with relative humidity.
- Adipic acid/ Diethylene Glycol/ Glycerin Crosspolymer; enhances film adhesion and film continuity with excellent film-forming properties.
- PVP/Eicosene copolymer; oil soluble polymer (alkylated polyvinylpyrrolidones) with high quality bioadhesive performance and excellent substantivity.

The matrices can be applied to the skin in the form of pellicule removal masks, metal ion scavenger cleansing creams, metal ion provider consistent creams or metal ions provider washing out masks.

The importance of metal ions for skin biology has been demonstrated time and again in situations where metal allergies have developed or when metal are required to react as cofactors in skin metabolism and defense. Heavy transition metals such as Ni, Cr, etc. and others are well known allergenes that eventually induce an immediate rush when exposed to sensitized people. The metal binding capacity of chitosan and its' derivatives and the fact that these chelators are brought into intimate contact by the described patent with the skin in an humid atmosphere, ensures the binding of such metals to the formulated matrix which is then removed together with the metals. On the other hand, many metals are essential for skin metabolism and skin defense. Besides of the earth alkaline metals such as Ca or Mg ions known as cofactors in cellular metabolism, transitions metals such as Zn, Mn, Cu, V, and others are gaining more and more importance as cofactors of enzymes engaged in the turnover of extracellular matrix such as metallo-proteinases and collagen-synthesizing enzymes, and of radical scavenging enzymes such as of the superoxide dismutase family, as well as metals having physiological effects in cosmetics such as those of Sr as an anti-itching compound and of Zr or Zr/ Aluminum complexes for antiperspirant activity. Deficiencies of such metals may well result in disturbed skin mechanism manifesting in unhealthy and/ or prematurely aging skin. The maintenance of a proper balance of these essential metals in skin is therefore necessary. This can be achieved with chitosan and its derivatives loaded with proper metal salts . Metal salts can be salts of strong acids such as chlorides, sulfates, etc. or of weak acids such as acetates, lactates, , etc. i, e, of physiological organic counterions.

Loading of chitosan and its derivatives is achieved by simple titration. Typically, a 0.1 - 20% solution of chitosan or of its derivative is titrated by a usually higher concentration of an aqueous solution of the metal salt commonly at neutral or slightly acidic pH. The newly formed metal salt precipitates upon metal binding and the termination of precipitation is also an indicator for having reached saturation. The amount necessary for chitosan complexation is thus determined for the specific metal and the chitosan or chitosan-derivative and the precipitated metal loaded metal complex harvested by suction filtration. This ratio can be maintained when producing higher quantities preferably 1 kg - 100 kg metal complex batchwise and by washing by resuspension in a smaller volume of water. These derivatives are then harvested by filtration, washed shortly and then dried or directly used for formulation. Alternatively the chitosan can be filtered and resuspended.

It is a very important feature of the matrices that the cosmetic active, i.e. the chitosan or chitosan-derivative based chelator, is retained in the polymer matrix during application. By occluding the evaporation of water from the skin the humid atmosphere favors hydrating conditions for and the flow of the metal ions, providing in the case of excess of metal ions on the surface a flux in the direction of the chelator, i.e. metal scavenging effect, and in case of an excess of metal ions in the preloaded chelator a flux in the direction of the skin, i.e. a metal supplementing effect. This application is comparable to that of directly or inversely acting face masks. Like in the case of face masks, the application, i.e. the ion flow, can be finished by lifting the polymer matrix or by removing the formulation.

In conclusion, this invention is to regulate the metal content in the skin and is applicable for the prevention of metal-based changes in the skin either by removal of metal ions or by supplementation by metal ions.

The following examples illustrate the invention in more detail.

### Example 1: Metal ions scavenger Cleansing Cream

| Composition | | |
|---|---|---|
| | INCI-Name | % w/w (as supplied) |
| Part A | PPG-15 Stearyl Ether | 3.00 |
| | Cyclomethicone and PPG-15 Stearyl Ether | 4.00 |
| | Caprylic/capric Triglycerides | 2.00 |
| | Behenyl Alcohol | 2.00 |
| | Isohexadecane | 4.00 |
| Part B | Sorbitan Stearate and Sucrose Cocoate | 3.00 |
| | Aqua | qsp |
| | Xanthan Gum | 0.25 |
| | Propylene Glycol | 4.00 |
| Part C | Diazolidinyl Urea/ Methyl paraben/ Propyl paraben/ Propylene Glycol | 0.70 |
| | Tocopheryl Linoleate | 0.30 |
| | Aqua | 10.00 |
| | Dicarboxymethylated Chitosan | 0.50 to 1.00 |
| Part D | Fragrance | qs |

### Preparation:

Phase B is prepared and heated to 80°C, cooled under moderate stirring to 75-70°C, then the Rhodicare S is dispersed.
At 75°C, part A is added into part B whilst stirring efficiently, homogenised at 10 000 rpm during 30 s, cooled under moderate stirring to 50°C and part C is added.

### Example 2:Metal ions Provider consistent cream

| Composition | | |
|---|---|---|
| | INCI-Name | % w/w (as supplied) |
| Part A | Glyceryl Stearate and PEG-100 Stearate | 3.50 |
| | Stearoyl Phytosphingosine | 0.35 |
| | Isohexadecane | 8.00 |
| | Potassium Cetyl Phosphate | 1.00 |
| | Cetyl Alcohol | 1.50 |
| | Octyldodecyl Myristate | 0.50 |
| | Isopropyl Isostearate | 2.00 |
| | Helianthus Annus Hybrid(Sunflower)Oil | 2.50 |
| | Persea Gratissima(Avocado)Oil | 2.50 |
| | PVP/Eicosene Copolymer | 1.50 |
| Part B | Carbomer | 0.50 |
| | Sorbeth-30 | 4.00 |
| | Aqua | qsp |
| Part C | Dniazolidiyl Urea/ Methyl paraben/ Propyl paraben/ Propylene Glycol | 0.70 |
| | Scleroglucan | 3.00 |
| | Aqua | qs |
| | Phosphono-methylated Chitosan with Mn²⁺Cl₂ | 0.50 to 1.00 |
| Part D | Triethanolamine | 0.50 |

### Preparation:

Phase A is prepared and heated to 75°C.
Carbomer is dispersed in the water from phase B, then Atlas G2330 is added and heated to 75°C.
Phase A is slowly poured into phase B whilst stirring quite efficiently, then homogenized at 10 000 rpm during 30 s, cooled down whilst stirring moderately and phase C is add near 55° -50°C.

### Example 3: Metal ions Provider Washing out Mask

| Composition | | |
|---|---|---|
| | INCI-Name | % w/w (as supplied) |
| Part A | Titanium Dioxide | 1.25 |
| | Kaolin | 10.00 |
| | Octyldodecanol | 5.00 |
| | Jojoba Oil | 4.00 |
| | Cetyl Alcohol | 2.00 |
| | Ceteareth-12 | 1.50 |
| | Ceteareth-20 | 2.50 |
| | Octylstearate | 3.00 |
| | Glyceryl Stearate | 1.25 |
| | Palmitic Acid | 1.25 |
| Part B | Magnesium Aluminium Silicate | 3.00 |
| | Aqua | qsp |
| Part C | Dniazolidiyl Urea/ Methyl paraben/ Propyl paraben/ Propylene Glycol | 0.80 |
| | Tocopheryl Linoleate | 0.30 |
| | Aqua | |
| | Phosphono-methylated Chitosan ZnCI | 0.50 to 1.00 |
| Part D | Sodium Hydroxide | qs |
| | Fragrance | qs |

### Preparation:

All ingredients (except Kaolin and Titanium Dioxide) of Phase A are added in a beaker, heated around 75°C and Kaolin and Titanium Dioxide are dispersed in it under moderate stirring.
Phase B is prepared by dispersing Veegum Ultra in the water and heated at 80°C during 10-15 min. under moderate stirring.
At about 80° -75°C, phase B is added into Phase A, homogenized 10 s at 10 000 rpm, cooled down under moderate stirring and phase C is incorporated near 50°C.

### Example 4: Metal ions Scavenger Pellicule removal Mask

| Composition | | |
|---|---|---|
| | INCI-Name | % w/w (as supplied) |
| Part A | Polyvinyl Alcohol | 12.00 |
| | Titanium Dioxide | 5.00 |
| | Hydrogenated Palm/Palm Kernel Oil PEG-6 Esters | 9.00 |
| | PEG-6 Caprylic/Capric Glycerides | 8.00 |
| | Aqua | qsp |
| | Adipic Acid/Diethylene Glycol/ Glycerin Crosspolymer | 3.00 |
| Part B | | |
| | Alcohol | 8.00 |
| | Butylene Glycol | 3.00 |
| | Magnesium Sulfate | 5.00 |
| | Diazolidinyl Urea/ Methyl paraben/ Propyl paraben/ Propylene Glycol | 0.70 |
| | Scleroglucan | 3.00 |
| | Dicarboymethylated Chitosan | 0.50 to 1.00 |
| Part C | Fragrance | qs |

### Preparation:

To prepare the Phase A polyvinylalcohol is dispersed in the water under moderate stirring and heated up to 80°C, cooled down under stirring and to 75°C, then the remaining ingredients are added.
At about 65-60°C, one to one and following the order the ingredients of the phase B are incorporated.

## Claims

1. A method for (a) the removal of contaminating metal ions from the skin or (b) supplying essential metal ions to the skin, comprising applying to the skin a composition comprising a cosmetically compatible polymer matrix in which (a) chitosan or chitosan derivatives or (b) chitosan or chitosan derivatives which are preloaded with essential metal ions are incorporated and wherein the corresponding polymer matrix is able to immobilize the chitosan and keep it in intimate contact with the skin.

2. A method according to claim 1 wherein the cosmetically compatible polymer matrix is in form of a, gel, cream, dispersion, emulsion, suspension, lotion, foam, mousse or mask.

3. A method according to claim 1 wherein the cosmetically compatible polymer matrix is a hydrogel.

4. A method according to claim 1 wherein the cosmetically compatible polymer matrix is a component of a patch.

5. A method for regulating the metal content of the skin comprising applying to the skin a composition according to claim 1.

6. Composition comprising a cosmetically compatible polymer matrix in which (a) chitosan or chitosan derivatives or (b) chitosan or chitosan derivatives which are preloaded with essential metal ions are incorporated and wherein the corresponding matrix is able to immobilize the chitosan and keep it in intimate contact with the skin.
